# EUROPÄISCHE PATENTSCHRIFT

(11) **EP 0 947 194 B1**
(45) Veröffentlichungstag und Bekanntmachung des Hinweises auf die Patenterteilung: **11.12.2002**
(21) Anmeldenummer: 99110836.6
(22) Anmeldetag: 17.08.1995
(51) Int. Cl.: A61K 31/385

(54) **Darreichungsformen enthaltend feste Salze von R-Thioctsäure mit verbesserter Freisetzung und Bioverfügbarkeit**
Dosage form comprising solid salts of R-alpha-lipoic acid with improved bioavailability
Forme de dosage à biodisponibilité améliorée comprenant des sels solides de la R-acide alpha-lipoique

(30) Priorität: 22.09.1994 DE 4433764
(43) Veröffentlichungstag der Anmeldung: 06.10.1999
(62) Teilanmeldung aus: 95112892.5
(73) Patentinhaber: VIATRIS GmbH & Co. KG, 60314 Frankfurt am Main (DE)
(72) Erfinder: Hettche, Helmut, Dr., 63128 Dietzenbach (DE); Rischer, Matthias, Dr., 60388 Frankfurt (DE); Sarlikiotis, Werner, Dr., 33615 Bielefeld (DE)
(74) Vertreter: Wibbelmann, Jobst, Dr., Dipl.-Chem.

(56) Entgegenhaltungen:
- EP-A- 0 427 246
- EP-A- 0 427 247
- EP-A- 0 530 446
- DE-A- 4 317 646

## Beschreibung

Thioctsäure (= α-Liponsäure) ist chemisch 1,2-Dithiacyclopentan-3-valeriansäure. Die Herstellung der freien R-Thioctsäure ist beispielsweise in der DE-OS 41 37 773 beschrieben.

Thioctsäure ist Bestandteil des Zellstoffwechsels und wird daher in vielen Pflanzen und tierischen Organismen gefunden. Sie wirkt als eines der Coenzyme bei der oxydativen Decarboxylierung von Pyruvat und anderen α-Ketosäuren. Thioctsäure wird seit längerer Zeit bei verschiedenen Erkrankungen eingesetzt, so unter anderem bei Lebererkrankungen, bei Leberschädigungen durch Pilzvergiftung sowie bei diabetischer und alkoholischer Polyneuropathie, einer mit Stoffwechselerkrankungen einhergehenden Veränderung peripherer Nerven.

Die vorliegende Erfindung betrifft Arzneimittelformulierungen enthaltend Thioctsäure oder feste Salze von Thioctsäure mit verbesserter Bioverfügbarkeit.

Die Erfindung bezieht sich nicht nur auf die racemische Form, sondern ebenso auf die reine (R)-beziehungsweise (S)-Thioctsäure sowie auf Gemische von (R)- und (S)-Thioctsäure mit beliebiger Zusammensetzung.

Von dem reinen optischen Isomeren der Thioctsäure (R- und S-Form, das heißt R-Thioctsäure und S-Thioctsäure) sind im Gegensatz zu dem Racemat das R-Enantiomer vorwiegend antiphlogistisch und das S-Enantiomer vorwiegend antinociceptiv wirksam, wobei die antiphlogistische Wirkung des R-Enantiomeren beispielsweise um einen Faktor 10 stärker ist als diejenige des Racemats.

Die antinociceptive (analgetische) Wirkung des S-Enantiomeren ist beispielsweise um einen Faktor bis 6 stärker als diejenige des Racemats.

Die Enantiomeren stellen daher im Vergleich zu dem Racemat sehr viel spezifischere und stärker wirksame Wirkstoffe dar.

Die Wirkungen sind in EP 427 246 und EP 427 247 sowie im GbM 90 17 987.0 und EP 530 446 beschrieben.

Eine Kombination von Thioctsäure mit Vitaminen ist in EP 572 922 beschrieben.

R,S-Thioctsäure hat einen Schmelzpunkt von 60,5°C. R-Thioctsäure hat einen Schmelzpunkt von 50,6 - 50,7°C. Beide sind bei 25°C in Wasser zu 12,14 mg/10 ml, in Methanol, Ethanol, Chloroform, Dimethylformamid und n-Octanol zu mehr als 1000 mg/10 ml löslich.

Oral anzuwendende Arzneiformen haben im Vergleich zu Parenteralia üblicherweise einen Preisvorteil, der sich günstig auf die Tagestherapiekosten auswirkt. Die bisherigen Darreichungsformen der Thioctsäure haben jedoch den Nachteil, eine relativ niedrige Bioverfügbarkeit aufzuweisen. Eine niedrige Bioverfügbarkeit bedeutet, daß bei peroraler Gabe der Darreichungsform im Blut eines Probanden oder Patienten im Vergleich zur intravenösen Gabe relativ wenig des unveränderten Wirkstoffs aufgefunden wird. Dies hat zur Folge, daß bei peroraler Gabe die Wirksamkeit der Medikation nicht das Ausmaß erreichen kann, wie es bei der intravenösen Gabe der Wirksubstanz der Fall ist. Es besteht daher die Aufgabe, Darreichungsformen zu entwickeln, die bei guter Lagerstabilität eine möglichst hohe Bioverfügbarkeit aufweisen.

Gegenstand der vorliegenden Erfindung sind daher Darreichungsformen, deren Bioverfügbarkeit gegenüber denen der bisherigen Darreichungsformen erhöht ist.

Nachteilig ist die schlechte Freisetzung des Wirkstoffes R-Thioctsäure aus den daraus hergestellten Darreichungsformen. Bei der Freisetzungsprüfung nach dem Deutschen Arzneibuch, 10. Ausgabe (Blattrührermethode) oder der USP XXII (mit Apparatus 2) ist die Freisetzung des Wirkstoffes aus Tabletten entsprechend Beispiel 2a bei Verwendung von 0,06 N HCl als Freisetzungsmedium bei 37°C wie folgt:
Nach 15 Minuten: 6%
Nach 30 Minuten: 9%
Zerfallzeit: < 2 min

Dies steht im Gegensatz zum Verhalten von Tabletten aus dem Racemat der Thioctsäure, die bei gleicher Zusammensetzung der Tabletten folgende Freisetzung des Wirkstoffes zeigen (Methode wie oben):
Nach 15 Minuten: 99%
Nach 30 Minuten: 100%
Zerfallzeit: 2,5 min

Überraschend zeigte es sich, daß bei Verwendung der festen Salze der R-Thioctsäure zur Herstellung der Tabletten entsprechend Beispiel 3 wieder gute Freisetzungswerte erhalten werden (Methode wie oben):
Nach 15 Minuten: 75%
Nach 30 Minuten: 88%
Zerfallzeit: < 1 min

Als Salzbildner kommen zum Beispiel übliche Basen beziehungsweise Kationen in Frage, die in der Salzform physiologisch verträglich sind. Beispiele hierfür sind: Alkali- oder Erdalkalimetalle, wie beispielsweise Natrium, Kalium, Calcium. Magnesium, Ammoniumhydroxid, basische Aminosäuren wie beispielsweise Ornithin, Cystin, Methionin, Arginin und Lysin, Amine der Formel N R1 R2 R3 ,worin die Reste R1, R2 und R3 gleich oder verschieden sind und Wasserstoff, C1-C4-Alkyl, wie beispielsweise Methyl, Ethyl, Propyl, Isopropyl, Butyl, Isobutyl ,tert.-Butyl, oder Cl-C₄-Oxyalkyl bedeuten, wie beispielsweise Mono- und Diethanolamin, 1-Amino-2-propanol, 3-Amino-1-propanol; Alkylendiamine mit einer Alkylenkette aus 2 bis 6-C-Atomen wie beispielsweise Ethylendiamin oder Hexamethylentetramin, gesättigte cyclische Aminoverbindungen mit 4-6 Ringkohlenstoffatomen wie beispielsweise Piperidin, Piperazin, Pyrrolidon, Morpholin; N-Methylglucamin, Kreatin und Trometamol

Die Salzbildner sind in den oben genannten Patentschriften bereits allgemein aufgeführt, ohne daß jedoch auf die Herstellung der Salze und deren besondere Eignung für die Herstellung von bestimmten Darreichungsformen eingegangen wird.

Flüssige Darreichungsformen mit dem Trometamol-, Lysin- und Ethylendiaminsalz der R-Thioctsäure sind in verschiedenen Patentanmeldungen beschrieben, beispielsweise in EP 427 246 EP 427 247, EP 530 446.

Hierbei handelt es sich jedoch ausnahmslos um Lösungen, bei denen das Salz durch Zusammengeben von R-Thioctsäure und der entsprechenden Base in Lösung gebildet wird. Das Salz selbst wird dabei nicht isoliert, tritt also in kristalliner Form nicht auf. Darüber hinaus wird regelmäßig die Base im Überschuß eingesetzt, so daß ein reines Salz nicht isolierbar wäre.

Salze von R-Thioctsäure mit L-Lysin und L-Arginin sind im spanischen Patent 313 056 erwähnt. Es fehlen jedoch Angaben zur Herstellung und Charakterisierung der Salze und der Hinweis auf die bessere Freisetzung und Bioverfügbarkeit des Wirkstoffes aus daraus hergestellten Darreichungsformen.

Darreichungsformen mit verbesserter Bioverfügbarkeit der racemischen Thioctsäure lassen sich dadurch erreichen, daß sie feste Salze der Thioctsäure statt der bisher eingesetzten freien Säure enthalten. Hier ist die Bioverfügbarkeit überraschenderweise gegenüber den Darreichungsformen mit der freien Thioctsäure erhöht. Als Salzbildner können auch hier die oben angegebenen Salzbildner fungieren. Darreichungsformen mit diesen festen Salzen zur peroralen Anwendung sind neu und bisher nicht beschrieben. Die Salzbildner sind zwar in verschiedenen Patentschriften bereits allgemein aufgeführt, ohne daß jedoch auf deren besondere Eignung zur Herstellung bestimmter Darreichungsformen eingegangen wird.

Als Darreichungsformen für die Salze der Thioctsäure können nicht nur Tabletten, Granulate, Inhalationspulver, Hartgelatinekapseln und Pellets aufgeführt werden, sondern auch Weichgelatinekapseln, Dosieraerosole, Suspensionen, Salben und Suppositorien, bei denen das Salz in einer Grundlage inkorporiert ist, beispielsweise in amphiphilen oder lipophilen Medien, Polyethylenglykol oder Treibmitteln.

Bei den letztgenannten Darreichungsformen kann im Vergleich zur Thioctsäure ebenfalls eine bessere Freisetzung aus der Grundlage beobachtet werden.

Gegenstand der Erfindung sind weiterhin Darreichungsformen, enthaltend feste Salze der R-Thioctsäure. Die Darreichungsformen können entweder als Arzneimittel oder als Lebensmittelzusatzstoff Verwendung finden. Die Salze können aus den oben beschriebenen Salzbildnern und R-Thioctsäure gebildet werden. Besonders bevorzugt sind die Salze aus R-Thioctsäure und Trometamol, L-Lysin, L-Arginin, Natriumhydroxid, Ammoniumhydroxid und Creatin. Die Herstellung erfolgt nach allgemein in der Technik bekannten Methoden, wie sie im Zusammenhang mit dem Racemat der Thioctsäure beispielsweise in DE-OS 16 95 358, dem französischen Patent 4630 M oder Beispiel 1 beschrieben sind.

Die analytischen Kennzahlen einiger der als besonders bevorzugt geltenden Salze sind:

| **Säure** | **Salzbildner** | **Schmelzpunkt** |
|---|---|---|
| R-Thioctsäure | Trometamol | 116 - 118 °C |
| | Natriumhydroxid | 247 - 257 °C (Zersetzung) |
| | ohne (freie Säure) | 50,6 - 50,7 °C |
| Racemische Thioctsäure | Trometamol | 120°C |
| | ohne (freie Säure) | 60,5°C |

Bei Einsatz von optisch aktiven Salzbildnern kann ein weiterer Vorteil genutzt werden:

Es ist möglich, racemische Thioctsäure mit dem optisch aktiven Salzbildner umzusetzen zu den R- und S-Thioctsäure-Salzen. Diese können aufgrund ihrer unterschiedlichen Eigenschaften getrennt und anschließend direkt zu den Darreichungsformen weiterverarbeitet werden, wobei sich eine Freisetzung der R- beziehungsweise S-Thioctsäure erübrigt. Als Salzbildner können beispielsweise eingesetzt werden: L-Lysin, L-Arginin, D(-)-N-Methylglucamin.

Die Herstellung der Darreichungsformen erfolgt nach den hierfür üblichen Standardmethoden, wie sie beispielsweise in dem Standardwerk Sucker, Fuchs, Speiser Pharmazeutische Technologie, Thieme Verlag Stuttgart, 1978, beschrieben sind.

Die Magensaftresistenz der Darreichungsformen wird entweder erzielt durch Einbettung des Wirkstoffs in anionische Polymere beispielsweise mit einem pKₛ-Wert von 5,0 bis 5,5 und nachfolgende Herstellung entsprechender Darreichungsformen unter Verwendung dieser Einbettung. Die übliche Methode zur Erzielung einer Magensaftresistenz von Formulierungen ist jedoch deren Überziehung mit anionischen Polymeren beispielsweise mit einem pKₛ-Wert von 5,0 bis 5,5, die sich erst bei einem pH-Wert von minimal 5,0 beginnen aufzulösen.

Als solche Substanzen wären beispielsweise zu nennen: Schellack Hydroxypropylmethylcellulosephthalat (Handelsprodukt beispielsweise HP 55/Hersteller: Shinetsu Chemical Company, Tokio), Celluloseacetatphthalat (Handelsprodukt beispielsweise Aquateric/Hersteller: FMC Export Corporation, Philadelphia, USA), Polyvinylacetatphthalat, Copolymerisate aus Methacrylsäure und Methacrylsäureestern (Handelsprodukte beispielsweise Eudragit L und Eudragit S-Typen sowie Eudragit L 30 D/Hersteller: Rhöm Pharma). Die Herstellung der Darreichungsformen erfolgt nach den hierfür üblichen Standardmethoden, wie sie beispielsweise in dem Standardwerk Sucker, Fuchs, Speiser Pharmazeutische Technologie, Thieme Verlag Stuttgart 1978, beschrieben sind sowie in Bauer, Lehmann, Osterwald Rothgang Überzogene Arzneiformen, Wissenschaftliche Verlagsgesellschaft mbH Stuttgart, 1988.

Die Prüfung auf Magensaftresistenz erfolgt nach der Methode der USP XXII, <701> Disintegration, wobei die Darreichungsformen eine Resistenz gegenüber künstlichem Magensaft über zwei Stunden zeigen müssen, mindestens jedoch über 30 Minuten.

### Beispiel 1:

### Synthese von Salzen der Thioctsäure

1 Äquivalent der Base wird in Ethanol suspendiert, auf ca. 50 °C erwärmt und unter Rühren mit 1 Äquivalent Thioctsäure versetzt. Anschließend wird noch kurz nachgerührt, die Lösung abgekühlt, wobei das Salz langsam auskristallisiert. Nach vollständiger Kristallisation wird das Salz abfiltriert und im Vakuum getrocknet.

### Vergleichsbeispiel:

### Vergleichsbeispiel: Tabletten mit 200 mg R-Thioctsäure

224,00 g R-Thioctsäure, 168,00 g Lactose, 83,40 g mikrokristalline Cellulose, 30,24 g Maisstärke und 10,08 g Poly(lvinyl-2-pyrrolidon) werden gesiebt, gemischt und mit gereinigtem Wasser befeuchtet. Anschließend wird das befeuchtete Gemisch über ein Lochsieb granuliert. Im Anschluß daran wird das feuchte Granulat im Wirbelschichtgranulator getrocknet. Danach werden 35,32 g mikrokristalline Cellulose, 2,24 g hochdisperses Siliciumdioxid und 6,72 g Magnesiumstearat gesiebt und mit dem trockenen Granulat vermischt. Die so entstandene Preßmasse wird mit Hilfe einer Tablettiermaschine zu Tabletten verpreßt.

Eine Tablette wiegt 500,0 mg und enthält 200,0 mg R-Thioctsäure.

### Beispiel 2

### Hartgelatinekapseln mit 158,7 mg Trometamolsalz der Thioctsäure, entsprechend 100 mg Thioctsäure

158,7 g Trometamolsalz der Thioctsäure werden mit 107,5 g mikrokristalliner Cellulose, 1,3 g hochdispersem Siliciumdioxid und 2,5 g Magnesiumstearat gemischt. Die Mischung wird zu jeweils 270 mg in Hartgelatinekapseln gefüllt.

### Beispiel 3

### Magensaftresistente Tabletten mit 221 mg Natriumsalz der Thioctsäure, entsprechend 200 mg Thioctsäure

221 g Natriumsalz der Thioctsäure, 100 g Lactose Monohydrat, 100 g mikrokristalline Cellulose, 21,5 g Maisstärke, 5 g Magnesiumstearat und 2,5 g hochdisperses Siliciumdioxid werden gemischt und die Mischung zu bikonvexen Tabletten vom Gewicht 450 mg und einem Durchmesser von 11 mm gepreßt.

2 g Hydroxypropylmethylcellulosephthalat (Handelsname: HP 55, Shinetsu) werden in einer Mischung von 20 g absolutem Ethanol und 30 g Methylenchlorid gelöst. Die Lösung wird in üblicher Weise in einem Wirbelschicht-Coater auf die Tabletten gesprüht, wobei der Coater mit einem geschlossenen Kreislauf zur Wiedergewinnung des Lösungsmittels und mit einer Inertisierung mittels Stickstoff versehen ist. Nach dem Trocknen werden die Tabletten entsprechend den Vorschriften der USP XXII auf Magensaftresistenz geprüft, wobei als Prüfflüssigkeit 0,1 N HCl bei 37°C eingesetzt wird. Mit der oben angegebenen Lösung werden die Tabletten so lange weiter besprüht und getrocknet, bis die Magensaftresistenz erreicht ist.

### Beispiel 4

### Magensaftresistente Tabletten mit 200 mg Thioctsäure

200 g Thioctsäure werden mit 100 g Lactose-Monohydrat, 100 g mikrokristalliner Cellulose , 20 g Maisstärke gemischt und die Mischung mit einer Lösung von 5 g Polyvidon K 25 in 60 g gereinigtem Wasser angefeuchtet. Falls erforderlich, wird zur Erzielung einer granulierfähigen Masse mit gereinigtem Wasser nachgefeuchtet und die erhaltene Masse durch ein Sieb der Maschenweite 2 mm granuliert. Nach dem Trocknen wird das Granulat durch ein Sieb der Maschenweite 1 mm gegeben und mit 5 g Magnesiumstearat sowie 2,5 g hochdispersem Siliciumdioxid gemischt. Die Mischung wird zu bikonvexen Tabletten mit einem Gewicht von 432,5 mg und einem Durchmesser von 11 mm gepreßt.

2 g Hydroxypropylmethylcellulosephthalat (Handelsname: HP 55, Shinetsu) werden in einer Mischung von 20 g absolutem Ethanol und 30 g Methylenchlorid gelöst. Die Lösung wird in üblicher Weise in einem Wirbelschicht-Coater auf die Tabletten gesprüht, wobei der Coater mit einem geschlossenen Kreislauf zur Wiedergewinnung des Lösungsmittels und mit einer Inertisierung mittels Stickstoff versehen ist.
Nach dem Trocknen werden die Tabletten entsprechend den Vorschriften der USP XXII auf Magensaftresistenz geprüft, wobei als Prüfflüssigkeit 0,1 N HCl bei 37°C eingesetzt wird. Mit der oben angegebenen Lösung werden die Tabletten so lange besprüht und getrocknet, bis die Magensaftresistenz erreicht ist.

### Beispiel 5

### Tabletten mit 663 mg Natriumsalz der Thioctsäure, entsprechend 600 mg Thioctsäure

1326 g Natriumsalz der Thioctsäure werden mit 200 g mikrokristalliner Cellulose, 52 g Maisstärke, 8 g Hochdispersem Siliciumdioxid und 14 g Magnesiumstearat gemischt und die Mischung zu Oblong-Tabletten vom Gewicht 800 mg verpreßt.
Die Tabletten können anschließend in üblicher Weise mit einem magensaftlöslichen oder magensaftpermeablen oder entsprechend Beispiel 13 mit einem magensaftresistenten Überzug versehen werden.

## Patentansprüche

1. Darreichungsformen zur peroralen Anwendung, enthaltend physiologisch verträgliche feste Salze von racemischer Thioctsäure, ausgenommen das Calciumsalz, das Aluminiumsalz sowie Aminosäuresalze in der Menge von 0,1 mg bis 250 mg.

2. Darreichungsformen nach Anspruch 1, die magensaftresistent sind.

3. Darreichungsformen nach Anspruch 1 oder 2, die Tabletten, Kapseln, Pellets, Dragees oder Granulate sind.

4. Darreichungsformen nach einem der Ansprüche 1 bis 3, die feste Salze von racemischer Thioctsäure mit einer Base, ausgewählt aus Alkali- oder Erdalkalimetallen, Ammoniumhydroxid, basischen Aminosäuren wie Ornithin, Cystin, Methionin, Arginin und Lysin, Aminen der Formel N R1 R2 R3, worin die Reste R1, R2 und R3 gleich oder verschieden sind und Wasserstoff, C1-C4-Alkyl oder Cl-C4-Oxyalkyl bedeuten, Alkylendiaminen mit einer Alkylenkette aus 2 bis 6-C-Atomen wie Ethylendiamin oder Hexamethylentetramin, Pyrrolidon, Morpholin; N-Methylglucamin, Kreatin und Trometamol, enthalten.

5. Darreichungsformen nach einem der Ansprüche 1 bis 4, die das feste Trometamolsalz von racemischer Thioctsäure enthält.

6. Darreichungsformen nach einem der Ansprüche 1 bis 5 zur Verwendung als Arzneimittel oder Lebensmittelzusatzstoff

## Claims

1. Administration forms for peroral use containing physiologically acceptable solid salts of racemic thioctic acid, except the calcium salt, the aluminium salt as well as amino acid salts in the amount of 0.1 mg to 250 mg.

2. Administration forms according to claim 1, which are resistant to gastric fluid.

3. Administration forms according to claim 1 or 2 which are tablets, capsules, pellets, dragees or granules.

4. Administration forms according to any of claims 1 to 3 containing solid salts of racemic thioctic acid with a base selected from alkali metals or alkaline earth metals, ammonium hydroxide, basic amino acids such as ornithine, cystine, methionine, arginine and lysine, amines of the formula N R₁ R₂ R₃, in which the radicals R₁, R₂ and R₃ are identical or different and denote hydrogen, C₁-C₄-alkyl or C₁-C₄-oxyalkyl, alkylenediamines with an alkylene chain of 2 to 6 C atoms such as ethylenediamine or hexamethylenetetraamine, pyrrolidone, morpholine; N-methylglucamine, creatine and trometamol.

5. Administration forms according to any of claims 1 to 4 containing the solid trometamol salt of racemic thioctic acid.

6. Administration forms according to any of claims 1 to 5 for use as pharmaceutical or food additive.

## Revendications

1. Formes d'administration pour l'utilisation perorale contenant des sels solides physiologiquement compatibles d'acide thiooctique racémique, à l'exception du sel de calcium, du sel d'ammonium ainsi que des sels d'aminoacides en quantité de 0,1 mg à 250 mg.

2. Formes d'administration selon la revendication 1, qui sont résistantes au suc gastrique.

3. Formes d'administration selon la revendication 1 ou la revendication 2, qui consistent en des comprimés, des capsules, des pellets, des dragées ou des granulés.

4. Formés d'administration selon l'une quelconque des revendications 1 à 3,
**caractérisées en ce qu'**
elles renferment des sels solides d'acide thiooctique racémique avec une base choisie parmi les métaux alcalins ou alcalino-terreux, l'hydroxyde d'ammonium, les aminoacides basiques comme l'ornithine, la cystine, la méthionine, l'arginine et la lysine, des aminés de formule, N R1 R2 R3 dans laquelle les radicaux R1 R2 et R3 sont identiques ou différents et signifient de l'hydrogène, un alkyle en C1-C4, ou un oxyalkyle en Cl-C4, des alkylènediamines ayant une chaîne alkylène à base de 2 à 6 atomes de carbone comme l'éthylènediamine ou l'hexaméthylènetétramine, la pyrolidone, la morpholine, la N-méthylglucamine, la créatine, et le trométamol.

5. Formes d'administration selon l'une quelconque des revendications 1 à 4,
**caractérisées en ce qu'**
elles renferment le sel solide de trométamol d'acide thiooctique racémique.

6. Formes d'administration selon l'une quelconque des revendications 1 à 5, en vue de l'utilisation en tant que médicament ou comme additif de produit alimentaire.
